# EUROPEAN PATENT SPECIFICATION

(11) **EP 3 266 443 B1**
(45) Date of publication and mention of the grant of the patent: **27.02.2019**
(21) Application number: 16178721.3
(22) Date of filing: 08.07.2016
(51) Int. Cl.: A61K 8/42, A61K 8/67, A61Q 19/00, A61K 8/92, A61K 8/04

(54) **TOPICAL MOISTURIZING COMPOSITION AND DISPENSER CONTAINING SAME**
TOPISCHE FEUCHTIGKEITSSPENDENDE ZUSAMMENSETZUNG UND SPENDER DAMIT
COMPOSITION HYDRATANTE TOPIQUE ET DISTRIBUTEUR LA CONTENANT

(43) Date of publication of application: 10.01.2018
(73) Proprietor: Omega Pharma Innovation & Development NV, 9810 Nazareth (BE)
(72) Inventor: DEW, Noel, 75225 Uppsala (SE); KRANTZ, Gustaf, 11251 STOCKHOLM (SE); HOLM, Tina, 12145 Johanneshov (SE)
(74) Representative: Brann AB

(56) References cited:
- DE-B1- 2 608 221
- JP-B2- 3 922 311
- GLOOR M A FLUHR J A LEHMANN L A GEHRING W A THIEROFF-EKERDT R B: "Do Urea/Ammonium Lactate Combinations Achieve Better Skin Protection and Hydration than Either Component Alone?", SKIN PHARMACOLOGY AND SKIN APPLIED PHYSIOLOGY, KARGER, BASEL, CH, vol. 15, 1 January 2002 (2002-01-01), pages 35-43, XP009185279, ISSN: 1422-2868
- GEHRING W: "Nicotinic acid/niacinamide and the skin", JOURNAL OF COSMETIC DERMATOLOGY, BLACKWELL SCIENCE, OXFORD, GB, vol. 3, no. 2, 1 January 2004 (2004-01-01) , pages 88-93, XP008080589, ISSN: 1473-2130, DOI: 10.1111/J.1473-2130.2004.00115.X
- BISSETT ET AL: "Common cosmeceuticals", CLINICS IN DERMATOLOGY, J.B. LIPPINCOTT, PHILADELPHIA, PA, US, vol. 27, no. 5, 1 September 2009 (2009-09-01), pages 435-445, XP026471172, ISSN: 0738-081X, DOI: 10.1016/J.CLINDERMATOL.2009.05.006 [retrieved on 2009-08-18]

## Description

### Field of the invention

The present invention relates to topical urea-containing moisturizers for moisturizing human skin, and more particularly a sprayable moisturizing low fat composition containing 2-9% of urea.

### Background of the invention

Urea is frequently used in topical moisturizing compositions, such as creams and lotions, for treating dry skin. Urea is e.g. known in the art to provide an improvement in skin barrier function under certain circumstances, see e.g. the paper by Marie Lodén, entitled "Urea-containing moisturizers influence the barrier properties of normal skin", Arch Dermatol Res (1996) 288: pp 103-107. See also the paper by M. Gloor, entitled ""Do Urea/Ammonium Lactate Combinations Achieve Better Skin Protection and Hydration than Either Component Alone?", Skin Pharma. and Skin Applied Physiology, (2002) 15: pp 35-43. Commercially available urea-containing moisturizers for treating dry skin typically exhibit a fat content of about 20% or more by weight of the composition, and a content of urea of typically no more than 5% by weight of the composition. Topical compositions having a fat content of about 20% are typically formulated so as to be rather viscous, such as creams and lotions, in order not to run, and are therefore not suitable for being applied directly on to the skin by means of spraying.

It is thus an object of the present invention to provide a topical moisturizing composition having a skin barrier improving function for easy, convenient application to the skin which also has favourable cosmetic properties derived from the low fat content.

### Summary of the invention

The above object has been accomplished by means of a topical moisturizing composition according to claim 1, which contains 2-9 % by weight of urea, 2-5 % by weight of niacinamide, and 5-10.5 % by weight of fat, wherein the combined amount of urea and nicinamide is at least 7 % by weight of the composition.

It has been found that an improvement in skin barrier function may be obtained with urea also at lower contents of fat. The inventive composition can therefore readily be made sprayable. According to the present invention a similar skin barrier improving function as with commercially available products can be obtained with the inventive low fat formulation.

The low fat content may also improve the cosmetic skin feel properties of the inventive composition.

According to the invention, the improvement in skin barrier function with urea at the inventive low fat content requires the presence of niacinamide (aka nicotinamide).

### Brief description of the drawings

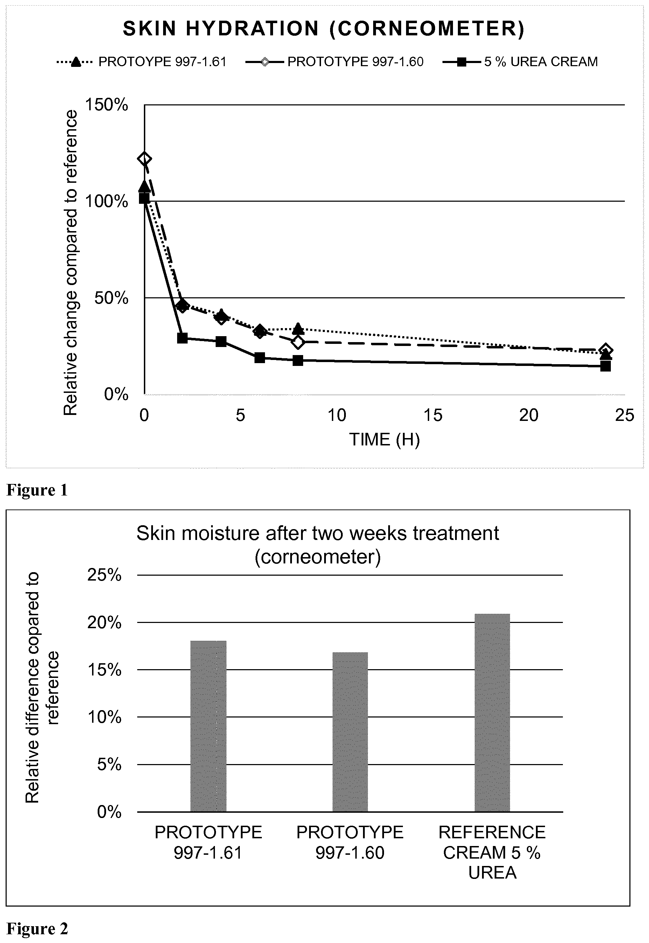

**Figure 1** shows skin moisture levels after application of inventive low fat (◇ and ▲) formulations, and a reference cream (■) having a 5 % or urea and conventional fat content. The moisture levels in the skin were increased for at least 24 hours after application of all formulas (n=20, p < 0.05).
**Figure 2** shows skin moisture levels after two weeks of twice daily application of two inventive low fat formulations (designated 997-1.61, and 997-1.60, respectively) and of the reference cream with the conventional higher fat level (n=17). All increases were significant (p < 0.05).

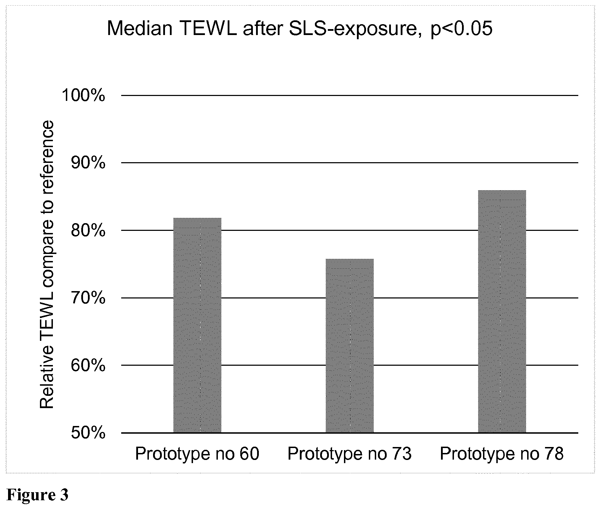

**Figure 3** shows the skin barrier function displayed by transepidermal water loss (TEWL) after sodium lauryl sulfate (SLS)-application on areas after two weeks of twice daily application of three low fat formulations (n=17) according to the invention. Treatments were compared to untreated skin which was considered as 100 %.

### Detailed description

The inventive topical composition is an oil-in-water emulsion wherein urea is dissolved and delivered to the skin via the water phase. The inventive composition is in general composed of water, light oil, humectant and emollients, urea, emulsifiers, preservatives and pH adjusting ingredients.

Niacinamide has been suggested to improve the skin barrier function under certain circumstances (Levin et al., J Clin Aesthet Dermatol. 2010 Feb; 3(2): 22-41). Accordingly, in the study by Tanno et al., Br J Dermatol. 2000;143:524-531, as referred to in the above review by Levin et al, it was concluded that nicotinamide improved the epidermal permeability barrier.

It has now surprisingly been found that, in the presence of niacinamide, urea will provide a skin barrier function in low fat formulations. Even more surprisingly, the niacinamide has been found only to provide a skin barrier improving function at low concentrations, i.e. up to 5 % by weight of the composition.

While the experiments presented herein have been conducted using 3 % and 5 % of niacinamide respectively, it is believed that the observed synergistic effect will also be achieved with a concentration of 2 % of niacinamide, as indicated to be effective by Tanno et al. in their *in-vivo* studies described in the above above.

According to the testing provided herein the observed inventive skin barrier strengthening effect seems to require a total amount of urea and niacinamide of at least 7 % by weight in the topical composition.

In its most general form the inventive topical composition comprises 2-9% by weight of urea, 2-5 % of niacinamide, and 5-10.5 % by weight of fat, wherein the combined amount of urea and niacinamide is at least 7 % by weight of the composition.

Overall the main components of the inventive composition typically are as follows:

| **Component** | **Weight % interval** |
|---|---|
| Water | Up to 100 |
| Humectants | 5-15 |
| Fats and oils | 5-10.5 |
| Urea | 2-9 |
| Niacinamide | 2-5 |
| Skin conditioning agents | 1-5 |
| Emulsifiers | 1-5 |
| Preservatives | 1-5 |
| pH adjusting ingredients | 0.1-1 |

### Fat

All fats and oils used in the inventive composition are fluid at room temperature. Oils used in the inventive composition are as follows.

| **Component (oil)** | **Weight % interval** |
|---|---|
| Light, e.g. ester, oils* | 1-10 |
| Natural triglyceride oils* | 1-5 |
| Mineral oil or silicone oils* | 0.1-5 |

| | |
|---|---|
| *All oils used in the formulation are of cosmetic grade or higher. | |

In preferred embodiments the fat comprises canola oil, preferably making out 20-50% by weight of the total fat.

### Urea

In preferred embodimets urea is contained in an amount of 2-7% by weight of the compostion, and more preferably in an amount up to 5% by weight.

### Additional additives

In order to stabilize the inventive formulation ingredients with various functionalities also may have to be added. Functionalities that may be crucial to formulation stability are preservatives, emulsifiers and to some extent pH adjusting ingredients. Some ingredients added may have multiple functions such as also providing skin conditioning or humectant functions as well as chelating of metal ions hence improving long term stability.

The additional functional ingredients used in the inventive composition can be summarized as follows:

| **Component*** | **Weight % interval** | **Function** |
|---|---|---|
| Emulsifyers | 1-5 | Emulisifier |
| Skin conditioners | 0.1-10 | Skin conditioning |
| Thickeners | 0.1-5 | Emulsifier/thickener |
| pH-adjusting or buffering ingredients | 0-5 | pH adjustment |
| Preservatives | 0.1-5 | Preservative |

| | | |
|---|---|---|
| *All ingredients used in the formulation are of cosmetic grade or higher. | | |

### Examples

In the following the invention will be further illustrated with reference to examples.

### Example 1

A formulation with desired properties was prepared having the following composition.

| ***INCI (UE)*** | ***% INCI*** |
|---|---|
| AQUA | 70.3 |
| GLYCERIN | 5.0 |
| UREA | 5.0 |
| ISOSTEARYL ISOSTEARATE | 3.0 |
| NIACINAMIDE | 3.0 |
| PROPYLENE GLYCOL | 3.0 |
| CANOLA OIL | 3.0 |
| DIMETHICONE | 2.0 |
| ISOPROPYL MYRISTATE | 2.0 |
| PHENOXYETHANOL | 1.0 |
| POLYGLYCERYL-6 STEARATE | 0.9 |
| ALLANTOIN | 0.5 |
| BUTYROSPERMUM PARKII EXTRACT | 0.5 |
| SODIUM LACTATE | 0.3 |
| ACRYLATES/C10-30 ALKYL ACRYLATE CROSSPOLYMER | 0.2 |
| POTASSIUM SORBATE | 0.1 |
| XANTHAN GUM | 0.1 |
| POLYGLYCERYL-6 BEHENATE | 0.1 |
| LACTIC ACID | <0.1 |
| CITRIC ACID | <0.1 |
| **Total** | **100.0** |

### Example 2

A formulation with desired properties was prepared having the following composition.

| ***INCI (UE)*** | ***% INCI*** |
|---|---|
| AQUA | 71.4 |
| GLYCERIN | 5.0 |
| UREA | 5.0 |
| ISOSTEARYL ISOSTEARATE | 3.0 |
| NIACINAMIDE | 3.0 |
| PROPYLENE GLYCOL | 3.0 |
| CANOLA OIL | 3.0 |
| ISOPROPYL MYRISTATE | 2.0 |
| POLYGLYCERYL-6 STEARATE | 1.8 |
| PHENOXYETHANOL | 1.0 |
| ALLANTOIN | 0.5 |
| DIMETHICONE | 0.5 |
| SODIUM LACTATE | 0.3 |
| ACRYLATES/C10-30 ALKYL ACRYLATE CROSSPOLYMER | 0.2 |
| POLYGLYCERYL-6 BEHENATE | 0.2 |
| POTASSIUM SORBATE | 0.1 |
| LACTIC ACID | <0.1 |
| CITRIC ACID | <0.1 |
| Total | **100.0** |

### Example 3

A formulation with desired properties was prepared having the following composition.

| ***INCI (UE)*** | ***% INCI*** |
|---|---|
| AQUA | 75.4 |
| GLYCERIN | 5.0 |
| NIACINAMIDE | 5.0 |
| PROPYLENE GLYCOL | 3.0 |
| ISOSTEARYL ISOSTEARATE | 2.0 |
| UREA | 2.0 |
| CANOLA OIL | 2.0 |
| POLYGLYCERYL-6 STEARATE | 1.8 |
| ISOPROPYL MYRISTATE | 1.0 |
| PHENOXYETHANOL | 1.0 |
| ALLANTOIN | 0.5 |
| DIMETHICONE | 0.5 |
| SODIUM LACTATE | 0.3 |
| ACRYLATES/C10-30 ALKYL ACRYLATE CROSSPOLYMER | 0.2 |
| POLYGLYCERYL-6 BEHENATE | 0.2 |
| POTASSIUM SORBATE | 0.1 |
| LACTIC ACID | <0.1 |
| CITRIC ACID | <0.1 |
| **Total** | **100.0** |

The above three formulations could readily be transferred into an aerosol.

A low viscosity composition with dry skin after-feel was prepared from fat, 5 % by weight of urea, and niacinamide.

The following study was conducted to investigate the barrier strengthening effect of the test formula. Twenty volunteers were needed for each test product were included in the study. The experiment was carried out as a randomized double-blinded study. The reference was untreated skin. No other skin care products were used on the forearms during the whole duration of the study. The study was proceeded with a three day wash-out period where no other products may be applied to the forearms, day 0-3. The volunteers were instructed to apply the test cream(s) on randomized predefined areas of their inside forearms twice daily, morning and evening for 2 weeks. The volunteers were instructed to use a defined amount of product. To assess the skin quality after two weeks of product application the TEWL and skin hydration was assessed on day 18. Prior to these measurements the volunteers washed their arms in the morning of the measurements with a mild soap and carefully rinsed this off with water, then pat dry the arms with a towel.

To assess the susceptibility to irritation, the treated and the untreated areas of the inside forearms were exposed to 50 µl of 1 % Sodium Lauryl Sulfate (SLS) in deionized water for 24 hours, applied in Finn Chambers on day 18. While the Finn Chambers were applied the volunteers avoided taking exercise and also used protective arm covers should they shower. The Finn Chambers were removed on day 19 and any SLS solution adhering to the skin was washed off with tap water and the arms pat dry with paper towels. The application areas were then marked with a permanent marker and the TEWL of each exposed site is measured 24 hours after the Finn chambers were removed on day 20.

### Results

The investigation showed that the inventive formulas with low fat content and improved cosmetic properties were as efficient in providing immediate moisture to the skin as the reference formulation with high fat content. The moisture levels were also increased for at least 24 hours after application of the formulation as can be seen in Figure 1. A two weeks long treatment period with twice daily application also increased the skin moisture level, as shown in Figure 2.

The skin barrier quality was evaluated after twice daily application for two weeks and it was found that several of the investigated inventive formulas with low fat content strengthened the skin as much as the reference formulation with higher fat content, which is displayed in Figure 3. The evaluation of the testing results shows that there is a barrier strengthening effect from formulas with fat content from 5-10 % and an urea content between 2-5 %. The barrier strengthening effect is dependent on presence of niacinamide which was added in an amount of 3 %, and 5 %, respectively, but no real benefits could not be seen with the higher amount added. However, when niaciamide was left out, no barrier strengthening effect was observed. The barrier strengthening effect is not seen at combined concentrations of urea and niacinamide lower than 7 % at fat a concentration of 5 %. It therefore seems that fat concentration is less important than the combined amount of urea and niacinamide and that the effect of the two latter ingredients is synergistic. Detailed results can be seen in table 1 below.

**Table 1. TEWL after two weeks of product use and one day of SLS exposure (n=17).**

| Formulation | 60 | 73 | 74 (Comparative) | 75 (Comparative) | 78 |
|---|---|---|---|---|---|
| Median | **0.8185** | **0.7582** | 0.9082 | 0.9741 | **0.8603** |
| p-value | **0.029** | **0.003** | 0.112 | 0.475 | **0.045** |
| Niacinamide | **3** | **3** | 3 | - | **5** |
| Urea | **5** | **5** | 2 | 5 | **2** |
| Fat | **10.5** | **8.5** | 5 | 8.5 | **5** |
| SheaTris | + | - | - | - | - |

## Claims

1. A topical moisturizing composition for application on human skin containing 2-9 % by weight of urea, 2-5 % by weight of niacinamide, and a total amount of fat of 5-10.5 % by weight of the composition, wherein the combined total amount of urea and niacinamide is at least 7 % by weight of the composition.

2. The topical moisturizing composition of claim 1, wherein the urea content is 2-5 % by weight, more preferably 3-5 % by weight of the composition.

3. The topical moisturizing composition of claim 1 or 2, wherein the niacinamide content is from about 3 to 5 % by weight, more preferably about 3 % by weight of the composition.

4. The topical moisturizing composition of any one of the previous claims, wherein the fat comprises canola oil, preferably making out 20-50 % by weight of the total fat content.

5. A dispenser for dispensing an aerosol, containing the composition of any one of the previous claims.

## Patentansprüche

1. Eine topische feuchtigkeitsspendende Zusammensetzung zur Anwendung auf der menschlichen Haut, die 2-9 Gew.-% Harnstoff, 2-5 Gew.-% Niacinamid und eine Gesamtmenge an Fett von 5-10,5 Gew.-% der Zusammensetzung enthält, wobei die kombinierte Gesamtmenge an Harnstoff und Niacinamid mindestens 7 Gew.-% der Zusammensetzung beträgt.

2. Die topische feuchtigkeitsspendende Zusammensetzung gemäß Anspruch 1, wobei der Harnstoffgehalt 2-5 Gew.-% beträgt, stärker bevorzugt 3-5 Gew.-% der Zusammensetzung.

3. Die topische feuchtigkeitsspendende Zusammensetzung gemäß Anspruch 1 oder 2, wobei der Niacinamidgehalt etwa 3 bis 5 Gew.-%, stärker bevorzugt etwa 3 Gew.-% der Zusammensetzung beträgt.

4. Die topische feuchtigkeitsspendende Zusammensetzung gemäß einem der vorherigen Ansprüche, wobei das Fett Rapsöl umfasst, das bevorzugt 20-50 Gew.-% des gesamten Fettgehalts ausmacht.

5. Ein Spender zum Dispergieren eines Aerosols, der die Zusammensetzung gemäß einem der vorherigen Ansprüche enthält.

## Revendications

1. Composition hydratante topique pour l'application sur la peau humaine contenant 2-9 % en poids d'urée, 2-5 % en poids de niacinamide, et une quantité totale de graisse de 5-10,5 % en poids de la composition, où la quantité totale combinée d'urée et de niacinamide est d'au moins 7 % en poids de la composition.

2. Composition hydratante topique selon la revendication 1, où la teneur en urée est 2-5 % en poids, de préférence encore 3-5 % en poids de la composition.

3. Composition hydratante topique selon la revendication 1 ou 2, où la teneur en niacinamide est d'environ 3 à 5 % en poids, de préférence encore d'environ 3 % en poids de la composition.

4. Composition hydratante topique selon l'une quelconque des revendications précédentes, où la graisse comprend de l'huile de colza, de préférence constituant 20-50 % en poids de la teneur en graisse totale.

5. Distributeur pour distribuer un aérosol, contenant la composition selon l'une quelconque des revendications précédentes.
